Europäisches Patentamt

European Patent Office

Office européen des brevets

(11) Publication number: **0 293 201**
**A1**

## EUROPEAN PATENT APPLICATION

(21) Application number: **88304784.7**

(22) Date of filing: **26.05.88**

(51) Int. Cl.⁴: **A 61 K 39/29**
**C 07 K 7/08, C 07 K 7/10,**
**C 12 N 15/00, A 61 K 37/02**

(30) Priority: **26.05.87 US 53892**

(43) Date of publication of application:
**30.11.88 Bulletin 88/48**

(84) Designated Contracting States:
**AT BE CH DE ES FR GB GR IT LI LU NL SE**

(71) Applicant: **THE WISTAR INSTITUTE**
**Thirty-Sixth Street at Spruce**
**Philadelphia Pennsylvania 19104-4268 (US)**

(72) Inventor: **Celis, Esteban**
**211 Martroy Lane Wallingford**
**Pennsylvannia (US)**

(74) Representative: **Bannerman, David Gardner et al**
**Withers & Rogers 4 Dyer's Buildings Holborn**
**London, EC1N 2JT (GB)**

A request for correction of the drawings has been filed pursuant to Rule 88 EPC. A decision on the request will be taken during the proceedings before the Examining Division (Guidelines for Examination in the EPO, A-V, 2.2).

(54) **Method of vaccination for hepatitis B virus.**

(57) A method is taught for vaccinating humans against hepatitis B infection in which a free polypolypeptide, corresponding to amino acid residues 14-33 of the hepatitis B surface antigen, is injected to induce immunity. T-cells which are specific for the hepatitis B surface antigen are stimulated to proliferate in response to the polypeptide.

EP 0 293 201 A1

## Description

## VACCINATION FOR HEPATITIS B VIRUS

Field of the Invention

This invention relates to hepatitis B virus infection, more particularly, it relates to the induction of immunity to the hepatitis B virus. Most particularly, it relates to the induction of a T-cell immune response to hepatitis B virus.

Background of the Invention

Hepatitis B infection of humans can cause a variety of diseases of the liver. These include acute and chronic hepatitis, cirrhosis, and heptocellular carcinoma. In some parts of the world, such as in parts of China, southern Asia, and tropical Africa the prevalence of hepatitis B infection is high. In addition, heptocellular carcinoma is one of the most common tumors found in humans, the great majority of it thought to be caused by the hepatitis B virus. Thus hepatitis is a world-wide health problem. (See Deinhardt et al. Journal of Medical Virology, vol. 17, pp. 209-217, 1985.)

While hepatitis B vaccines exist, they are expensive and the protection they provide is not always complete. (See Linneman et al., The Lancet, vol. i, pp. 346-47, 1984 and Purcell, Hepatology, vol. 5, pp. 159-63, 1985). Most vaccines which are currently in use are produced from pooled plasma of individuals who are positive for hepatitis B surface antigen. However, recombinant vaccines have recently been approved for use which can be produced in yeast cell as well as prokaryotes or other eukaryotes. (See Purcell, supra.) A live vaccine containing DNA fragments of hepatitis B antigen inserted into vaccinia virus is also in development. Many workers have also used synthetic polypeptides, corresponding to portions of the hepatitis B surface antigen and the hepatitis B core antigen to immunize animals against hepatitis B virus.

Almost all of the work directed toward defining which synthetic polypeptides are immunogenic has been focused on the induction of a B-cell or antibody response. Some workers have attempted to define synthetic polypeptides which would induce a T-cell response to hepatitis B virus. (Milich et. al. Journal of Immunology, vol. 134, pp. 4203-11, 1985). However, this work was done in mice, a species which cannot be infected by hepatitis B virus. The present invention demonstrates that this is not an analogous animal system to humans for hepatitis B infection, as different synthetic polypeptides are found to be immunogenic in the mouse and human systems.

SUMMARY OF THE INVENTION

It is an object of the present invention to provide a method for inducing a T-cell immune response to hepatitis B virus in humans.

It is an object to the present invention to provide a synthetic polypeptide which is capable of inducing immunity to hepatitis B virus in humans.

It is an object of the present invention to provide a vaccine for hepatitis B virus, the source of which is not dependent upon plasma from chronically infected individuals.

It is still another object of the present invention to provide a hepatitis B vaccine which can augment the immune response induced by antibody-inducing vaccines.

In accordance with this invention there is provided a method of immunizing a human against hepatitis B virus, comprising injecting into a human a free polypeptide comprising a portion of the hepatitis B surface antigen, said portion comprising amino acid residues 14 to 33 of hepatitis B surface antigen encoded by the S gene of hepatitis B virus.

In another embodiment of the present invention the human being immunized is also injected with a conventional vaccine comprising killed hepatitis B virus, hepatitis B surface antigen, or a synthetic polypeptide which stimulates the production of hepatitis B-specific antibodies. The present invention also provides biologically pure preparations comprising a polypeptide having a sequence corresponding to hepatitis B surface antigen selected from the group consisting of amino acid residues 14 to 33 and 4 to 33.

The polypeptides of the present invention provide less expensive and more efficacious means of preventing hepatitis B infections. In addition, they eliminate the possibility of contaminants being administered to recipients of vaccines derived from plasma donors.

BRIEF DESCRIPTION OF THE DRAWINGS

Figure 1 shows two of the sequences of hepatitis B surface antigen which were synthesized.

Figure 2 depicts a map of the hepatitis B surface antigen and peptides corresponding to it which were tested for T-cell stimulating activity.

Figure 3 shows cytotoxic activity of human T cells to target cells expressing hepatitis B antigenic determinants.

Figure 4(a) shows the response of hepatitis B-specific human T cells to the presence of hepatitis B related antigens. Figure 4(b) shows the response of rabies virus-specific human T Cells to the same antigens, as well as to rabies as a positive control.

DETAILED DESCRIPTION OF THE INVENTION

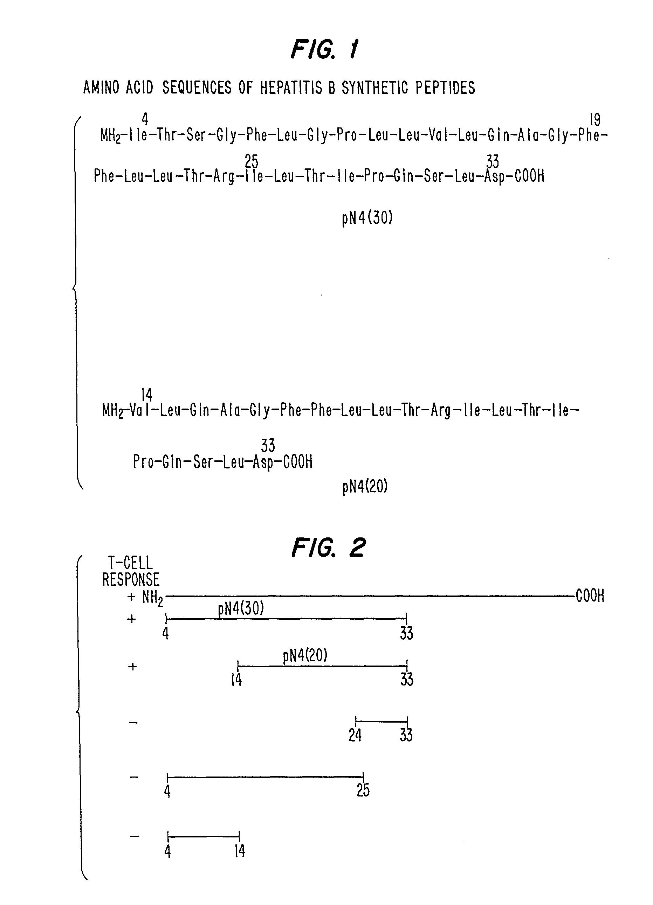

In accordance with the present invention it has been discovered that a synthetic polypeptide having a sequence which corresponds to amino acid residues 14 through 33 of the hepatitis B surface antigen encoded by the S gene of hepatitis B virus is able to induce a proliferative response in human T-cells. A thirty amino acid-long polypeptide comprising residues 4 through 33 has been shown to have some T-cell stimulating activity, and a shorter polypeptide of twenty amino acid residues comprising amino acids 14 through 33 shows similar level of activity. The exact amino acid sequences which were synthesized are shown in Figure 1.

The gene coding for the surface antigen of hepatitis B virus has previously been cloned and completely sequenced for about six strains. (Tiollais et al. "Structure and Organization of Hepatitis B virus DNA." In: Verme et. al., eds., Viral hepatitis and delta infection. Proc. of Int'l Symp. on Viral Hepatitis. New York: Alan R. Liss, Inc. 1983, pp 11-22.) The surface antigen encoded by the S gene is 226 amino acid residues and is made up of alternating regions of hydrophobic and hydrophilic characters. It had been postulated that the hydrophilic regions represent the most antigenic regions of the molecule because they would most likely be on the surface of the virion. The hydrophobic regions have been postulated to be less antigenic presumably because they are thought to be embedded in the lipid portion of the viral coat. Dreesman et. al. Nature, vol. 295, pp. 158-160, 1982. Surprisingly, the polypeptide provided by the present invention is derived from one of the regions of the surface antigen which is hydrophobic.

According to the method of the present invention, free polypeptides may be used to immunize humans against hepatitis B virus. That is to say, that the polypeptides need not be chemically coupled to another protein or other polymeric substance in order to be antigenic for T cells. The capacity of free polypeptides to induce a T-cell immune response contrasts with results of others in which free synthetic polypeptides have been unable to induce an antibody response. Neurath et al., J. Gen. Virol., vol, 65, pp. 1009-14, 1984. In those cases, in order to induce an antibody response the synthetic polypeptides had to be coupled to other proteins, such as keyhole limpet hemocyanin. The present invention provides biologically pure preparations of a free polypeptide having the sequences of amino acid residues 14 through 33 of hepatitis B surface antigen. These preparations surprisingly have biological activity as antigens even in their pure, free state. Free polypeptides, in the present invention, refers to polypeptides which are not coupled to other proteins or erythrocytes or other polymers. However, the free polypeptides ovalently linked to other synthetic polypeptides, for example, a polypeptide which stimulates an antibody response. Thus, a single polypeptide could stimulate both T and B cell responses to hepatitis B virus. Free polypeptides can also be contained within liposomes.

Larger polypeptides having the amino acid sequences of residue 14-33, for example, a polypeptide comprising amino acid residues 4 through 33, are also operative in the present invention, although being more expensive to synthesize. Other-sized polypeptides containing amino acid residues 14 through 33 may also be used. Because the minimum amino acid residue requirements for T-cell stimulation has not been determined, it is also possible that less than all of the residues between 14 and 33 may be functional. Figure 2 summarizes what is known as to mapping the required amino acids for T-cell stimulation.

Although the sequence of the polypeptide described herein is described with complete particularity as to sequence, it is to be understood that this region of the hepatitis B surface antigen isolated from another strain might have a slightly variant sequence, although substantially equivalent function. It is thus intended that all polypeptides having substantially similar sequences to the one defined herein are encompassed by the present invention. Similarly, single amino acid changes which are innocuous to the function described herein are not considered beyond the scope of the present invention. For example, it is possible that less than all of amino acid residues 14 to 33 are necessary such that a peptide having residues 15-32 may still be a potent T-cell stimulator. Similarly, substitution of a residue which is not critical for antigenicity may still result in an active peptide. Likewise a conservative amino acid change from one of the residues shown to a similar amino acid may result in an antigenic peptide.

Means other than synthetic chemistry can be used to generate these polypeptides, and such other means as known in the art are also contemplated by the present invention. For example, enzymatic cleavage of larger protein fragments can be used to generate the polypeptides of the present invention. Alternatively, the polypeptides can be synthesized by an in vitro translation system, such as the rabbit reticulocyte system. In yet another way of preparing the polypeptides of the present invention, a coupled transcription-translation system can be used. In still another contemplated means of generating the polypeptides of the present invention, a fragment of the hepatitis B S gene can be cloned and inserted into an appropriate cell line. Thus, the polypeptide can actually be synthesized in vivo and isolated for further purification.

According to the method of the present invention, injection of the polypeptide vaccines is accomplished according to routine and well known methods in the art. Thus injection may be subcutaneous or intramuscular, for example. Similarly, when a polypeptide of the present invention is used to stimulate a T-cell response and to augment the response of a conventional vaccine, such conventional vaccine is administered in the routine and known way in the art. Such conventional vaccines can comprise for example, killed hepatitis B virus, synthetic peptides, hepatitis B surface antigen, or other antigens which stimulate an antibody response to hepatitis B virus. The advantage of using the two-step vaccination procedure is that the antibody responses to hepatitis B surface antigen would be greater since helper T lymphocytes would be primed by the prior administration of the peptide of the present invention. The vaccines may be formulated in carriers as are known in the art. Most likely these will be sterile aqueous solutions but other carriers may be desired. Likewise preservatives or stabilizers may be used so long as the antigenicity of the preparation is not diminished. The dosage ranges for administration are those large enough to produce the desired T-cell proliferation effect. The dosage should not be so large as to produce adverse side effects. Dosage determinations are within the still of those of ordinary skill in the art. Most likely, appropriate dosages will be in the range of about 50-100 ug per injection. Of course, as is known to those of skill in the art, this will vary with the age, size and general condition of the patient.

The following examples are provided to illustrate the present invention, which is defined by the specification and claims as a whole. The examples are not intended to limit the scope of the invention, but are included for illustrative purposes only.

#### Example 1

Ten polypeptides corresponding to 80 percent of the hepatitis B surface antigen molecule were synthesized by solid-phase chemistry following the technique described by Merrifield, Adv. Enzymol., vol. 32, pp. 221-296, 1969. The polypeptides were purified and their amino acid composition determined; each polypeptide was found to be more than 90 percent pure. The polypeptides corresponds to the following amino acid residues of the hepatitis B surface antigen encoded by the S gene: 4-33, 34-53, 54-73, 64-112, 93-112, 113-132, 136-155, 163-192, 202-222, 212-222.

The capacity of each of the ten synthetic polypeptides to stimulate the proliferative response of human T-cell lines specific for hepatitis B surface antigen was determined. A long-term, cultured T-cell line with helper activity, isolated from a hepatitis B virus vaccinee, was initially used. Only one of the ten polypeptides synthesized potently induced a proliferative response. This polypeptide corresponded to the amino terminal end of the hepatitis B surface antigen molecule, from amino acid residue 4 to residue 33. This antigenic polypeptide, called pN4(30) was able to stimulate a proliferative response in other T-cell clones isolated from the same vaccinee. Further, this same polypeptide pN4(30) was able to stimulate T-cells from three other vaccinees. In control experiments the pN4(30) was used to stimulate T-cells specific for other antigens, such as rabies virus; no proliferative response was triggered. Isolation of the hepatitis B surface antigen-specific T cell lines is described in Celis et al., Journal of Immunology vol. 132, pp. 1511-1516 (1984).

The synthetic peptide found herein to be able to stimulate T-cell proliferation is distinct from that found by Milich et. al. for mouse T-cells, which corresponded to residues 38-56.

#### Example 2

In order to map the precise location of the antigenic determinant contained within pN4(30), a series of smaller polypeptides were prepared and tested. A polypeptide which contains the twenty amino acids of the carboxyl-terminal portion of pN4(30), i.e., amino acid residues 14 to 33, was found to stimulate effectively the hepatitis B surface antigen-specific T cells. This twenty amino acid polypeptide is called pN4(20). Three other polypeptides were found to be incapable of stimulating the hepatitis B surface antigen-specific T cells. These inactive polypeptides correspond to the amino acid residues of the hepatitis B surface antigen of: 24-33, 4-25, and 4-14. A map showing these results is depicted in Figure 2.

#### Example 3

This example shows that cells expressing hepatitis B surface antigen or pN4(20) antigenic determinants are killed by hepatitis B surface antigen-specific human T cells.

Target cells were prepared by incubating B lymphoblastoid cells overnight with 100 ug/ml antigenic determinants, either hepatitis B surface antigen or pN4(20). The target cells were washed two times by centrifugation to remove unincorporated antigenic determinants. The target cells were then incubated with $^{51}$Cr. (Methods as described in McMichael et al., J. Gen. Virology Vol. 67, pp. 719-726, and Townsend et al. Cell, vol. 44, pp. 959-968, 1986.)

Aliquots of target cells were incubated with varying numbers of effector cells, i.e., hepatitis B specific T cells. The effector cells were prepared by the method of Celis, supra. After a 5 hour incubation, the amount of $^{51}$Cr released with the medium was determined.

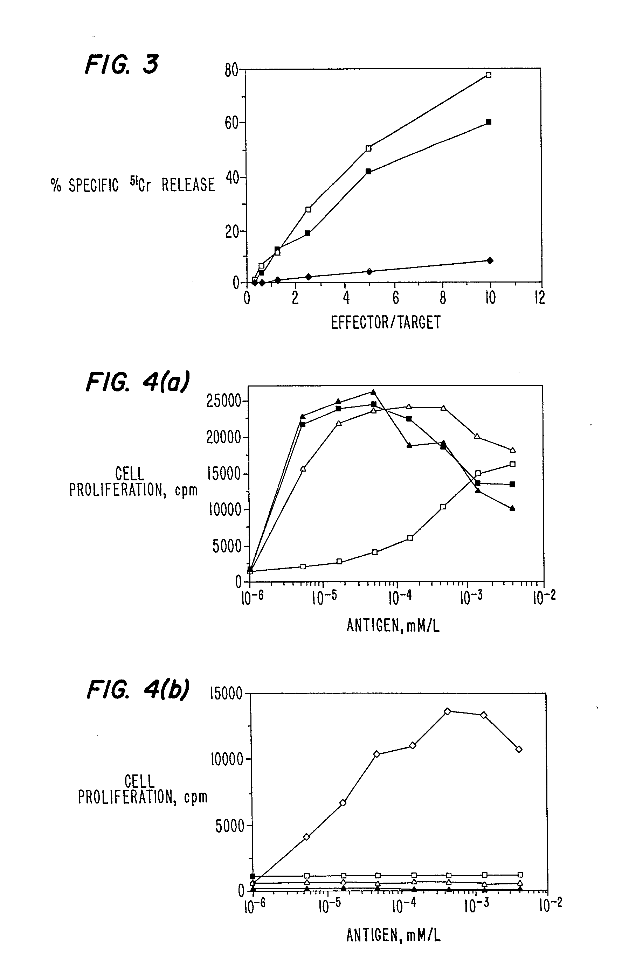

The results are shown in Figure 3. The amount of $^{51}$Cr released into the medium represents the amount of cytolytic activity of the effector cells. The lymphoblastoid cells were labeled with hepatitis B surface antigen (■), pN4(20) (□), or medium above (♦). Target cells not expressing a hepatitis B surface antigen-related polypeptide were not efficiently killed by the hepatitis B surface antigen specific T cells.

#### Example 4

The relative capacities of the two polypeptides pN4(20) and pN4(30) and hepatitis B surface antigen, native and denatured, to induce the proliferative response of a human hepatitis B surface antigen-specific T cell line was examined.

Denatured hepatitis B surface antigen was prepared by resuspending purified hepatitis B surface antigen in 70% formic acid for 30 minutes in ice. The formic acid was evaporated under vacuum and the antigen was resuspended in culture medium. A constant number of T cells ($2 \times 10^4$) was incubated with different molar concentrations of the antigens in the presence of antigen presenting cells for three days at 37°C under tissue culture conditions. The cultures were pulsed with tritiated thymidine during the last 18 hours of incubation, to determine the degree of cell proliferation.

Figure 4a shows the T cell proliferation stimulated by each of the antigen preparations at various concentrations, as represented by the amount of tritiated thymidine incorporated into the cells (counts per minute or cpm). As can readily be noted, both of the synthetic polypeptides pN4(20) (■) and pN4(30) (▲), induced similar degrees of T cell proliferation. Similar results were observed with the denatured form of hepatitis B surface antigen (△). On the other hand, the native or unmodified hepatitis B surface antigen (□) was 500-1000 times less potent than the other antigens in inducing the proliferation of the T cells at certain concentrations of antigen.

Figure 4b shows a similar experiment but using a rabies virus-specific human T cell line. In this case none of the hepatitis B surface antigens (or peptides) induced a proliferative response. Rabies antigens (◊) stimulated the proliferation of this cell line. These results indicate that the stimulatory activity of peptides pN4(20) and pN4(30) is antigen specific towards T cells reactive to hepatitis B surface antigen.

**Claims**

1. A biologically pure preparation comprising a free polypeptide having the sequence corresponding to amino acid residues 14-33 of hepatitis B surface antigen encoded by the S gene of hepatitis B virus.

2. A biologically pure preparation comprising a free polypeptide having the sequence corresponding to amino acid residues 4-33 of hepatitis B surface antigen encoded by the S gene of hepatitis B virus.

3. A biologically pure preparation comprising a free polypeptide having a sequence selected from the group of those shown in Figure 1.

## FIG. 1

AMINO ACID SEQUENCES OF HEPATITIS B SYNTHETIC PEPTIDES

$MH_2$-Ile-Thr-Ser-Gly-Phe-Leu-Gly-Pro-Leu-Leu-Val-Leu-Gin-Ala-Gly-Phe-

Phe-Leu-Leu-Thr-Arg-Ile-Leu-Thr-Ile-Pro-Gin-Ser-Leu-Asp-COOH

pN4(30)

$MH_2$-Val-Leu-Gin-Ala-Gly-Phe-Phe-Leu-Leu-Thr-Arg-Ile-Leu-Thr-Ile-

Pro-Gin-Ser-Leu-Asp-COOH

pN4(20)

## FIG. 2

**FIG. 3**

% SPECIFIC $^{51}Cr$ RELEASE

EFFECTOR/TARGET

**FIG. 4(a)**

CELL PROLIFERATION, cpm

ANTIGEN, mM/L

**FIG. 4(b)**

CELL PROLIFERATION, cpm

ANTIGEN, mM/L